# EUROPÄISCHE PATENTSCHRIFT

(11) **EP 4 100 101 B1**
(45) Veröffentlichungstag und Bekanntmachung des Hinweises auf die Patenterteilung: **23.09.2026**
(21) Anmeldenummer: 21705440.2
(22) Anmeldetag: 05.02.2021
(51) Int. Cl.: B29C 45/00, A61M 39/24, B29C 67/24

(54) **RÜCKSCHLAGVENTIL, TROPFKAMMER, PORT ZUM NADELLOSEN ZUDOSIEREN EINER FLÜSSIGKEIT, RÜCKFLUSSSPERRE, INFUSIONS- ODER TRANSFUSIONSSYSTEM UND VERFAHREN ZUM HERSTELLEN EINES RÜCKSCHLAGVENTILS**
NON-RETURN VALVE, DRIP CHAMBER, PORT FOR NEEDLE-FREE METERING OF A LIQUID, BACK-FLOW BARRIER, INFUSION OR TRANSFUSION SYSTEM AND METHOD FOR PRODUCING A NON-RETURN VALVE
CLAPET DE NON-RETOUR, CHAMBRE DE GOUTTE-À-GOUTTE, ORIFICE POUR LE DOSAGE SANS AIGUILLE D'UN LIQUIDE, BARRIÈRE ANTI-REFLUX, SYSTÈME DE PERFUSION OU DE TRANSFUSION ET PROCÉDÉ DE FABRICATION D'UN CLAPET DE NON-RETOUR

(30) Priorität: 06.02.2020 DE 102020201483
(43) Veröffentlichungstag der Anmeldung: 14.12.2022
(73) Patentinhaber: B. Braun Melsungen AG, 34212 Melsungen (DE)
(72) Erfinder: SCHLITT, Christof, 34621 Frielendorf (DE)
(74) Vertreter: DREISS Patentanwälte PartG mbB
(86) Internationale Anmeldenummer: PCT/EP2021/052809
(87) Internationale Veröffentlichungsnummer: WO 2021/156440

(56) Entgegenhaltungen:
- EP-A1- 1 892 034
- WO-A1-00/57941
- WO-A1-2006/118748
- GB-A- 2 387 333
- US-A- 6 165 168
- US-B1- 6 537 258

## Beschreibung

Die Erfindung betrifft ein Rückschlagventil, eine Tropfkammer für ein Infusions- oder Transfusionssystem, einen Port zum nadellosen Zudosieren einer Flüssigkeit in ein Infusions- oder Transfusionssystem, eine Rückflusssperre für die Fluidleitung eines Infusions- oder Transfusionssystems, ein Infusions- oder Transfusionssystem, das das Rückschlagventil aufweist, und ein Verfahren zum Herstellen eines Rückschlagventils.

In der Medizintechnik spielen für verschiedene Therapiefelder und für verschiedene Arzneimittelverabreichungsmethoden Rückschlagventile eine wichtige Rolle. Beispielsweise kontrollieren Rückschlagventile bei der Infusions- oder Schmerztherapie die Belüftung von Tropfkammern, die Flussrichtung von Flüssigkeiten oder dienen als
Port (d.h. Zugang) zum nadellosen Zudosieren von Flüssigkeiten. Insbesondere wenn das Medizinprodukt zum einmaligen Gebrauch ausgelegt ist, sind niedrige Herstellkosten der verschiedenen Komponenten des Produkts wichtig, damit dieses wirtschaftlich vermarktet werden kann.

GB 2 387 333 A offenbart ein Rückschlagventil gemäß dem Oberbegriff von Anspruch 1. WO 2006/118748 A1 offenbart normal geschlossene, außenliegende Schlitzventile für medizinische Flüssigkeitsströme. EP 1 892 034 A1 offenbart ein Verfahren zur Herstellung einer Formmasse aus einer Rohmaterialschmelze, die mit einem Additiv beladen wird, US 6 165 168 A offenbart einen Adapter für Katheter. WO 00/57941 A1 offenbart eine einheitliche geformte Elastomerleitung zur Verwendung mit einer medizinischen Infusionspumpe. US 6 537 258 B1 offenbart ein Anti-Siphon-Ventil, für 2 medizinische Infusionsleitungen.

Verschiedene Rückschlagventile sind marktgängig und in der Literatur beschrieben. Für den Einsatz in der Medizintechnik ist es erforderlich, dass die Materialien, aus denen die Rückschlagventile bestehen, biokompatibel sind. Ferner müssen sie mit den Fluiden, mit denen sie bei der Verwendung in Kontakt kommen, kompatibel sein. Darüber hinaus müssen sie die erforderlichen mechanischen Eigenschaften aufweisen, beispielsweise ausreichende mechanische Festigkeit.

Rückschlagventile aus elastischen und biokompatiblen Materialien können eine entsprechende Eignung aufweisen. Es sind verschiedene Varianten bekannt. Beispielsweise beschreibt EP 0 515 597 B1 ein Scheibenventil, welches durch seine Bauform eine gewisse Vorspannung besitzt und hierdurch in eine Flussrichtung den Fluiddurchgang verschließt. Liegt allerdings ein bestimmter Druck, ausgelöst durch ein strömendes Fluid, aus der Gegenrichtung vor, hebt die Ventilscheibe ab und gibt den Durchgang frei. Die Herstellung von Scheibenventilen ist jedoch aufwendig und daher teuer. Insbesondere sind die Fertigungstoleranzen an die Ventilscheibe und den Ventilsitz in den Komponenten, in die das Ventil verbaut wird, sowie die Montagetoleranzen dieser Komponenten zueinander gering, was die Herstellung der Teile unwirtschaftlich macht. Aufgrund der hohen Anforderungen an die Toleranzen neigen Scheibenventile schon bei geringfügigen Maßabweichungen, die auch bei sorgfältiger Produktion nicht sicher ausgeschlossen werden können, dazu, Fluide in der Sperrrichtung durchzulassen. Solche Ventile sind daher nicht ausreichend sicher. Ferner sind so genannte Entenschnabel-Ventile bekannt, welche die Form eines Rohres aufweisen, welches sich an einem Ende entlang der Querachse linienförmig verjüngt, so dass nur ein Spalt verbleibt. Wird durch Fluidströmungen durch das Rohr ein Druck auf den Spalt ausgeübt, öffnet dieser. Bei Strömungen in Gegenrichtung sollte dieser hingegen verschlossen sein. Auch die Herstellung von Entenschnabel-Ventilen ist aufwendig und teuer. Entenschnabel-Ventile weisen zudem sehr hohe Leckraten auf, da der Spalt immer etwas offen steht und bei niedrigen Drücken in Sperrrichtung nicht geschlossen wird. Außerdem arbeiten sie bezüglich der Öffnungsdrücke sehr inkonstant.

Des Weiteren ist in der DE 82 26 186 U1 ein Topfventil aus einem elastischen Material beschrieben, bei welchem der topfförmige Ventilkörper nahe am Topfboden einseitig durchschnitten ist, d.h. einen Schlitz aufweist, sodass sich der Topfboden bis zu einem gewissen Grad vom übrigen Ventilkörper abheben kann. So wird der Durchgang durch den Druck eines durch den Topf strömenden Fluids geöffnet, jedoch bei Strömung in Gegenrichtung verschlossen. Die Funktion des Topfventils setzt die Herstellung aus einem elastischen Material voraus.

An das elastische Material wird die Anforderung gestellt, dass sich der eingeschnittene Schlitz im Zuge der Herstellung des Ventils und des Medizinprodukts sowie während des Lebenszyklus des Medizinprodukts bis zu dessen Haltbarkeitsdatum nicht wieder verschließt, etwa durch Chemikalien, wie beispielsweise zu Sterilisationszwecken eingesetztes Ethylenoxid, oder durch eingebrachte Energie in Form von Wärme oder anderer Strahlung, beispielsweise einer zu Sterilisationszwecken eingebrachten energiereichen Strahlung (Gammastrahlung etc.). Die Eigenschaft, dass sich der eingeschnittene Schlitz wieder zumindest teilweise verschließt, wird als "Selbstheilung" oder "Self-healing" des Materials bezeichnet. Unter einem teilweisen oder vollständigen Verschließen des Schlitzes wird dabei verstanden, dass die Schnittflächen sich verkleinern oder ganz verschwinden, weil das Material an der einen Schnittfläche sich mit dem Material an der anderen Schnittfläche verbindet, indem das Material an der einen Schnittfläche und das Material an der anderen Schnittfläche aneinander haften oder einander durchdringen. Eine derartige Selbstheilung führt dazu, dass das Rückschlagventil nicht oder zumindest nicht über ausreichend lange Zeit zuverlässig arbeitet.

Im Fall typischer Anwendungen eines Bauteils aus einem elastischen Material stellt die Selbstheilungstendenz des elastischen Materials kein Problem dar, unter Umständen ist die Selbstheilung herkömmlich sogar erwünscht. Dies kann daran liegen, dass das Bauteil keinen Bedingungen ausgesetzt ist, die die Selbstheilung fördern, also nicht durch entsprechende Chemikalien oder entsprechende Strahlung belastet wird. Daher wird der Selbstheilungstendenz eines elastischen Materials üblicherweise keine Bedeutung beigemessen. Der Erfinder hat dessen ungeachtet erkannt, dass die Selbstheilung insbesondere im Fall von Rückschlagventilen für medizinische Anwendungen, welche einen geschlitzten elastischen Ventilkörper aufweisen, unter Umständen nachteilig sein kann.

Herkömmliche Topfventile sind aus Materialien hergestellt, die diese Anforderungen nicht in zufriedenstellender Weise erfüllen, d.h. in einem nicht akzeptablen Ausmaß Selbstheilung zeigen, und/oder sie sind mittels aufwendiger und daher teurer Herstellungsverfahren hergestellt und/oder sie sind mittels langsamer und daher teurer Herstellungsverfahren hergestellt und/oder sie weisen eine geringe Bruchdehnung auf, die bedingt, dass der Topfboden an der Stelle des eingeschnittenen Schlitzes bei der Weiterverarbeitung des Ventils leicht abbrechen kann. Herkömmliche Topfventile bestehen beispielsweise aus HCR-Silikon. Die Abkürzung "HCR" steht für "High Consistency Rubber". Zur Herstellung eines Bauteil aus HCR-Silikon wird eine pastöse oder teigartige Masse, welche neben dem Kunststoffmaterial und etwaigen Füll- und Hilfsstoffen einen Vulkanisierer umfasst, in eine Form gepresst und in der Form bei hoher Temperatur quervernetzt. Dieses Verfahren wird auch als "HCR-Pressen" bezeichnet. HCR-Silikone sind nicht nur aufwendig in der Verarbeitung, sondern ergeben außerdem Bauteile mit geringer Bruchdehnung.

Eine Aufgabe der Erfindung besteht daher darin, ein verbessertes Rückschlagventil, insbesondere ein verbessertes Rückschlagventil für ein Medizinprodukt bereitzustellen. Speziell besteht eine Aufgabe der Erfindung darin, ein Rückschlagventil bereitzustellen, welches eine niedrige Leckrate aufweist und über einen langen Lebenszyklus zuverlässig arbeitet und welches dennoch auf einfache und wirtschaftlich günstige Weise hergestellt ist. Eine Aufgabe der Erfindung besteht ferner darin, verbesserte Medizinprodukte, die ein Rückschlagventil oder mehrere Rückschlagventile aufweisen, sowie Komponenten für derartige Medizinprodukte bereitzustellen.

Diese Aufgaben werden gelöst durch das Rückschlagventil gemäß Anspruch 1, die Tropfkammer gemäß Anspruch 9, den Port gemäß Anspruch 10, die Rückflusssperre gemäß Anspruch 11, das Infusions- oder Transfusionssystem gemäß Anspruch 12 und das Verfahren gemäß Anspruch 13. Dabei können die in Unteransprüchen und nachfolgender Beschreibung angeführten Merkmale auch zur Ausgestaltung des Gegenstands einer anderen Anspruchskategorie oder eines weiteren Patentanspruchs derselben Kategorie verwendet werden.

Bei dem erfindungsgemäßen Rückschlagventil handelt es sich um ein Rückschlagventil, das einen topfförmigen Ventilkörper umfasst. Der Ventilkörper weist eine Mantelwand, eine Bodenwand und eine der Bodenwand gegenüberliegende Öffnung auf. Der Ventilkörper umfasst ein elastisches Material. Das elastische Material ist entlang eines Schnitts durchschnitten und weist dadurch einen Schlitz auf.

Das elastische Material umfasst ein durch Flüssigspritzgießen unter Verwendung von Flüssigsilikonkautschuk verarbeitetes Material. Der Schnitt ist in der Mantelwand angeordnet ist, und ist
- entweder parallel zur Innenfläche der Bodenwand, oder nicht parallel zur Innenfläche der Bodenwand geneigt und weist dabei eine Neigung zur Innenfläche der Bodenwand auf, die maximal 10°, vorzugsweise maximal 5°, vorzugsweise maximal 2°, insbesondere maximal 1° beträgt.

Durch die Erfindung kann beispielsweise ein Rückschlagventil bereitgestellt werden, welches geringe Leckraten aufweist. Es kann bei unterschiedlichen Druckverhältnissen zuverlässig arbeiten und einen Fluss eines Fluids entgegen der Sperrrichtung verhindern. Es kann diese Eigenschaften über eine langen Lebenszyklus beibehalten. Dabei ist es fertigungstechnisch vergleichsweise einfach, die Anforderungen an die Toleranzen einzuhalten.

Durch die Erfindung kann beispielsweise ein bei Weiterverarbeitung in Folgeprozessen, Lagerung und Transport robustes Rückschlagventil bereitgestellt werden, das für die medizinische Verwendung geeignet ist. Ferner kann die Möglichkeit bestehen, das Rückschlagventil in Großserienfertigung herzustellen.

Darüber hinaus ist das Rückschlagventil mittels eines wirtschaftlich vorteilhaften Verfahrens hergestellt, was insbesondere für die Massenfertigung vorteilhaft ist. Es ist im Stand der Technik weder bekannt noch vorherzusehen gewesen, dass sich ein Rückschlagventil mit derartig vorteilhaften Eigenschaften auf eine wirtschaftlich derartig vorteilhafte Weise herstellen lässt.

Bei der erfindungsgemäßen Tropfkammer handelt es sich um eine Tropfkammer für ein Infusions- oder Transfusionssystem. Die Tropfkammer weist ein Belüftungsventil auf. Als Belüftungsventil dient ein erfindungsgemäßes Rückschlagventil. Dadurch wird eine Tropfkammer bereitgestellt, welche die Vorteile des erfindungsgemäßen Rückschlagventils hat.

Bei dem erfindungsgemäßen Infusions- oder Transfusionssystem gemäß einer Alternative handelt es sich um ein Infusions- oder Transfusionssystem mit mindestens einer Tropfkammer, wobei als Tropfkammer eine erfindungsgemäße Tropfkammer dient. Dadurch wird ein Infusions- oder Transfusionssystem bereitgestellt, welches die Vorteile des erfindungsgemäßen Rückschlagventils hat.

Im Rahmen dieser Offenbarung wird unter einem Infusions- oder Transfusionssystem ein System verstanden, mit dem die Verabreichung einer medizinischen Infusion oder die Durchführung einer medizinischen Transfusion vorgenommen werden kann. Insbesondere kann es sich um ein Infusionsset (auch als "Infusionsbesteck" bezeichnet) handeln. Ein Infusionsset weist einen Infusionsschlauch und einen Anschluss auf, mit dem der Infusionsschlauch an einen Behälter, der die einem Patienten oder einer Patientin zu verabreichenden Flüssigkeit beinhaltet, angeschlossen werden kann. Zwischen dem Anschluss und dem Infusionsschlauch ist optional eine Tropfkammer angeordnet. Bei dem Behälter kann sich beispielsweise um eine Infusionsflasche, einen Infusionsbeutel, eine Blutkonserve etc. handeln. Der Infusionsschlauch weist einen Anschluss für einen Patientenzugang (z.B. Venenkanüle oder Venenkatheter) auf. Der Patientenzugang kann optional als Teil des Infusionssets betrachtet werden. Das Infusionsset kann optional weitere Komponenten umfassen, beispielsweise einen Durchflussregler zur Kontrolle der Fließgeschwindigkeit der Flüssigkeit wie etwa eine Rollenklemme.

Bei dem erfindungsgemäßen Port handelt es sich um einen Port zum nadellosen Zudosieren einer Flüssigkeit in ein Infusions- oder Transfusionssystem, wobei als Port ein erfindungsgemäßes Rückschlagventil dient. Dadurch wird ein Port bereitgestellt, welcher die Vorteile des erfindungsgemäßen Rückschlagventils hat.

Bei dem erfindungsgemäßen Infusions- oder Transfusionssystem gemäß einer Alternative handelt es sich um ein Infusions- oder Transfusionssystem mit mindestens einem Port zum nadellosen Zudosieren einer Flüssigkeit, wobei als Port ein erfindungsgemäßer Port dient. Dadurch wird ein Infusions- oder Transfusionssystem bereitgestellt, welches die Vorteile des erfindungsgemäßen Rückschlagventils hat.

Bei der erfindungsgemäßen Rückflusssperre handelt es sich um eine Rückflusssperre für die Fluidleitung eines Infusions- oder Transfusionssystems, wobei als Rückflusssperre ein erfindungsgemäßes Rückschlagventil dient. Dadurch wird eine Rückflusssperre bereitgestellt, welche die Vorteile des erfindungsgemäßen Rückschlagventils hat.

Bei dem erfindungsgemäßen Infusions- oder Transfusionssystem gemäß einer Alternative handelt es sich um ein Infusions- oder Transfusionssystem mit mindestens einer Rückflusssperre zum Verhindern eines Flusses eines Fluids in einer Fluidleitung in einer Fließrichtung, wobei als Rückflusssperre eine erfindungsgemäße Rückflusssperre dient. Dadurch wird ein Infusions- oder Transfusionssystem bereitgestellt, welches die Vorteile des erfindungsgemäßen Rückschlagventils hat. Bei dem erfindungsgemäßen Verfahren handelt es sich um eine Verfahren zum Herstellen eines Rückschlagventils, welches einen topfförmigen Ventilkörper mit einer Mantelwand, einer Bodenwand und einer der Bodenwand gegenüberliegenden Öffnung aufweist, wobei das Verfahren die folgenden Schritte umfasst:
(A) Bereitstellen eines elastischen Materials,
(B) Flüssigspritzgießen eines elastischen Materials zum Herstellen des Ventilkörpers,
(C) Durchschneiden des elastischen Materials entlang eines Schnitts zum Erzeugen eines Schlitzes im Ventilkörper,
wobei der Schnitt in der Mantelwand angeordnet ist, und
- entweder parallel zur Innenfläche der Bodenwand ist, oder nicht parallel zur Innenfläche der Bodenwand geneigt ist und dabei eine Neigung zur Innenfläche der Bodenwand aufweist, die maximal 10°, vorzugsweise maximal 5°, vorzugsweise maximal 2°, insbesondere maximal 1° beträgt.

Das Bereitstellen des elastischen Materials kann dabei beispielsweise in Form von Komponenten erfolgen, aus deren Gemisch das elastische Material gebildet wird.

Weitere Merkmale und Zweckmäßigkeiten der Erfindung werden nachfolgend anhand von exemplarischen Ausführungsbeispielen des erfindungsgemäßen Rückschlagventils unter Bezugnahme auf die angeschlossenen Zeichnungsfiguren beschrieben. Durch den Einbau dieser Rückschlagventile in entsprechende Medizinprodukte ergeben sich erfindungsgemäße Ausführungsbeispiele dieser Medizinprodukte.
Fig. 1 zeigt eine isometrische Ansicht des erfindungsgemäßen Rückschlagventils gemäß einem ersten Ausführungsbeispiel der Erfindung.
Fig. 2 zeigt eine Schnittansicht des erfindungsgemäßen Rückschlagventils gemäß dem ersten Ausführungsbeispiel.
Fig. 3 zeigt eine weitere Schnittansicht des erfindungsgemäßen Rückschlagventils gemäß dem ersten Ausführungsbeispiel.

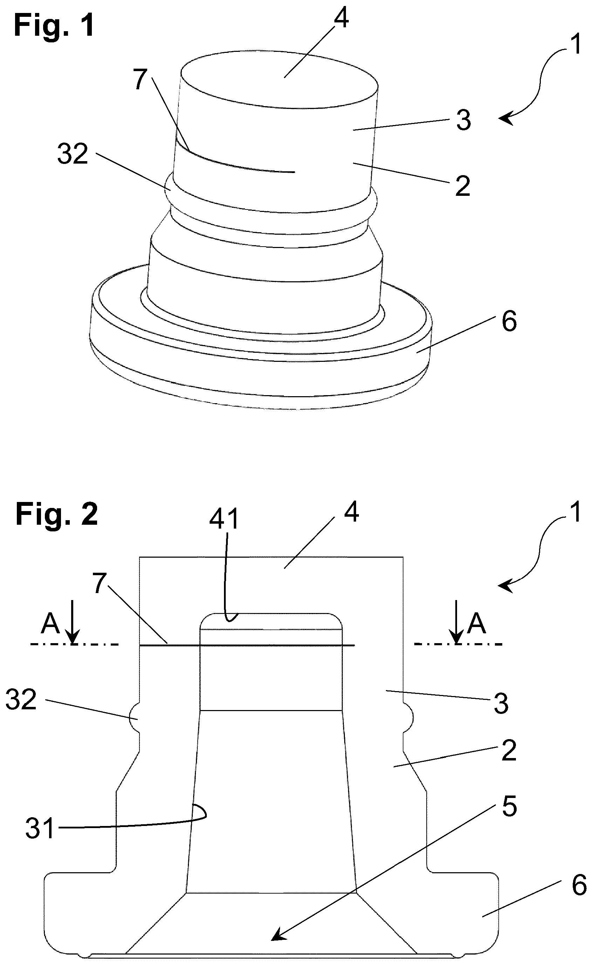

In den Figuren 1 bis 3 ist das erfindungsgemäße Rückschlagventil 1 in verschiedenen Ansichten dargestellt. Das Rückschlagventil 1 umfasst einen topfförmigen Ventilkörper 2. Das heißt der Ventilkörper 2 ist in Form eines einseitig geschlossenen Hohlkörpers ausgebildet. Der Ventilkörper 2 weist eine Mantelwand 3, die den Mantel des Hohlkörpers bildet, und eine Bodenwand 4, die den geschlossenen Boden des Hohlkörpers bildet, auf. Auf der der Bodenwand 4 gegenüberliegenden Seite weist der Ventilkörper 2 eine Öffnung 5 auf.

Um die Öffnung 5 herum ist auf der der Bodenwand 4 gegenüberliegenden Seite ein Flansch 6 angeformt. Der Flansch 6 dient der Befestigung des Rückschlagventils 1 in einem Bauteil, insbesondere in einer Komponente eines Medizinprodukts. Der Flansch 6 ist einstückig an die Mantelwand 3 angebunden. In alternativen, nicht in den Zeichnungsfiguren dargestellten Ausführungsbeispielen der Erfindung ist der Flansch 6 nicht einstückig mit dem übrigen Ventilkörper 2 ausgebildet, sondern als separates Element ausgebildet und mit der Mantelwand 3 verbunden. In weiteren alternativen, ebenfalls nicht in den Zeichnungsfiguren dargestellten Ausführungsbeispielen der Erfindung verfügt der Ventilkörper 2 über keinen Flansch 6 und ist dazu bestimmt, durch eine andere Befestigung als mittels eines Flansches in einem Bauteil befestigt zu werden, beispielsweise durch Kleben und/oder Schweißen und/oder eine Pressverbindung. Da der Flansch 6 eine sichere und einfache Befestigungsmöglichkeit für das Rückschlagventil 1 bereitstellt, ist es bevorzugt, dass das Rückschlagventil 1 einen Flansch 6 aufweist. Hinsichtlich Herstellung sowie Eigenschaften und Funktion des einstückig angeformten Flanschs 6 ist das Flüssiggießverfahren besonders vorteilhaft. Der Flansch 6 lässt sich mittels Flüssiggießverfahren ohne zusätzlichen Arbeitsschritt verwirklichen. Im Falle eines auf Materialabtragung beruhenden Herstellungsverfahrens müsste der Flansch 6 in einem separaten Schritt erzeugt werden, z.B. indem seine Form aus dem Ventilkörper 2 herausgeschnitten wird.

Die Mantelwand 3 ist in der in den Figuren 1 und 2 gezeigten Orientierung des Rückschlagventils 1, in der sich die Bodenwand 4 oben befindet, unterhalb der Innenfläche 41 der Bodenwand 4 einseitig durchschnitten, d.h. sie weist einen Schnitt 7 auf. Das Material der Mantelwand 3 ist entlang des Schnitts 7 durchschnitten.

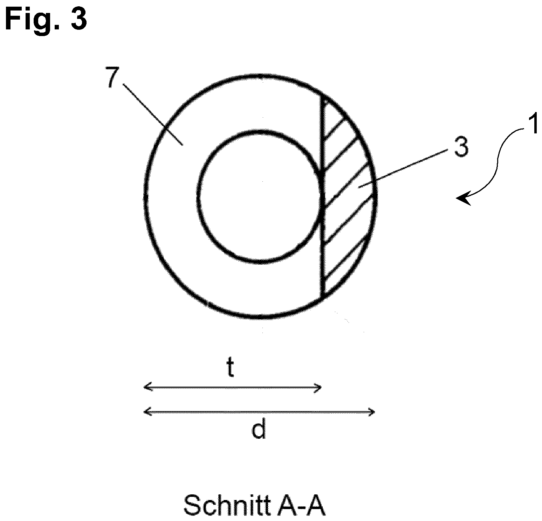

Fig. 3 zeigt einen Schnitt in der Ebene A-A, die in Fig. 2 gekennzeichnet ist und die senkrecht auf die Längsrichtung des Rückschlagventils 1 von der Öffnung 5 zur Bodenwand 4 steht. Der Schnitt 7 liegt in der Ebene A-A.

Im vorliegenden Ausführungsbeispiel liegt der Schnitt 7 demnach in einer Ebene, d.h. die Schnittflächen sind eben. In alternativen, nicht in den Zeichnungsfiguren dargestellten Ausführungsbeispielen liegt der Schnitt 7 nicht in einer Ebene, d.h. die Schnittflächen sind nicht eben sondern beispielsweise gewölbt.

Im vorliegenden Ausführungsbeispiel ist der Schnitt 7 parallel zur Innenfläche 41 der Bodenwand 4. In alternativen, nicht in den Zeichnungsfiguren dargestellten Ausführungsbeispielen ist der Schnitt 7 nicht parallel zur Innenfläche 41 der Bodenwand 4, sondern zur Innenfläche 41 der Bodenwand geneigt. Dabei ist es bevorzugt, dass der Schnitt (7) derart in der Mantelwand (3) angeordnet ist, dass er eine Neigung zur Innenfläche (41) der Bodenwand (4) aufweist, die maximal 10°, mehr bevorzugt maximal 5°, noch mehr bevorzugt maximal 2° und insbesondere bevorzugt maximal 1° beträgt.

Im vorliegenden Ausführungsbeispiel weist der Schnitt 7 einen Abstand zur Innenfläche 41 der Bodenwand 4 auf, der ca. 25% des Außendurchmessers d der Mantelwand 3 im Bereich des Schnitts 7 beträgt. In alternativen, nicht in den Zeichnungsfiguren dargestellten Ausführungsbeispielen weist der Schnitt 7 einen größeren oder kleineren Abstand von der Bodenwand 4 auf.

Die Tiefe t des Schnitts 7 beträgt im vorliegenden Ausführungsbeispiel 75% des Außendurchmessers d der Mantelwand 3 in der Ebene A-A, d.h. die Mantelwand 3 ist entlang 75% des Außendurchmessers durchtrennt und entlang der verbleibenden 25% nicht durchtrennt. Der nicht durchtrennte Bereich der Mantelwand 3 ist in Fig. 3 schraffiert dargestellt. In alternativen, nicht in den Zeichnungsfiguren dargestellten Ausführungsbeispielen ist der Schnitt 7 tiefer oder weniger tief als 75% des Außendurchmessers d der Mantelwand 3. Dabei ist es bevorzugt, dass die Tiefe t des Schnitts mindestens 50%, mehr bevorzugt mindestens 60%, noch mehr bevorzugt mindestens 70% und insbesondere bevorzugt mindestens 72,5% des Außendurchmessers d der Mantelwand 3 im Bereich des Schnitts 7 beträgt. Dabei ist es ferner bevorzugt, dass die Tiefe t des Schnitts höchstens 90%, mehr bevorzugt höchstens 85%, noch mehr bevorzugt höchstens 77,5% des Außendurchmessers d der Mantelwand 3 im Bereich des Schnitts 7 beträgt. Die Tiefe t des Schnitts 7 ist in allen Fällen so zu wählen, dass sich das Rückschlagventil 1 bei dem gewünschten Öffnungsdruck aber nicht bei niedrigeren Drücken zuverlässig öffnet, indem sich die Mantelwand im Bereich des Schlitzes öffnet und ein Fluid in der Durchlassrichtung hindurchströmen lässt.

Im vorliegenden Ausführungsbeispiel verjüngt sich die Innenfläche 31 der Mantelwand 3 in der Richtung von der Öffnung zur Bodenwand 4. In alternativen, nicht in den Zeichnungsfiguren dargestellten Ausführungsbeispielen ist die Innenfläche 31 der Mantelwand 3 kreiszylinderförmig.

Die Mantelwand 3 weist auf ihrer Außenseite eine umlaufende Wulst 32 auf. Diese Wulst 32 bietet fertigungstechnische Vorteile, beispielsweise da sie die Befestigung des Rückschlagventils in einem anderen Bauteil, das eine entsprechende Nut aufweist, verbessern kann. Außerdem kann die Wulst 32 die Abdichtung gegenüber diesem Bauteil verbessern, selbst wenn dieses keine Nut aufweist. In alternativen, nicht in den Zeichnungsfiguren dargestellten Ausführungsbeispielen weist die Mantelwand 3 alternativ oder zusätzlich zu dieser Wulst eine umlaufende Nut auf. Die Wulst oder Nut kann auch an der Innenfläche der Mantelwand 3 angeordnet sein. Die Wulst oder Nut muss nicht notwendigerweise umlaufend sein, d.h. entlang des gesamten Umfangs der Mantelwand 3 verlaufen. Es können auch mehrere Wulste und/oder Nuten vorhanden sein.

Im vorliegenden Ausführungsbeispiel ist das Rückschlagventil 1 abgesehen vom Schnitt 7 rotationssymmetrisch aufgebaut. Das ist für die Funktion des Rückschlagventils 1 nicht erforderlich, sodass auch andere Formen möglich sind. Eine rotationssymmetrische Form ist allerdings in vielen Fällen fertigungstechnisch vorteilhaft und/oder an die räumlichen Gegebenheiten des Bauteils, in das das Rückschlagventil 1 einzubauen ist, besser angepasst als beispielsweise eine Form mit einem elliptischen Querschnitt.

Der Ventilkörper 2 besteht im vorliegenden Ausführungsbeispiel aus einem elastischen Material. In alternativen Ausführungsbeispielen besteht der Ventilkörper nicht zur Gänze aus einem elastischen Material, sondern umfasst neben dem elastischen Material weitere Komponenten aus einem anderen Material, beispielsweise kann ein Flansch 6 aus einem nicht elastischen Material mit dem aus einem elastischen Material hergestellten übrigen Ventilkörper verbunden sein.

Der Ventilkörper 2 umfasst in jedem Fall ein elastisches Material, das durch Flüssigspritzgießen verarbeitet worden ist. Durch die Erfindung ist es somit möglich, ein Rückschlagventil 1 mit entsprechenden Eigenschaften herzustellen und dabei die Vorteile des Flüssigspritzgießens zu nutzen, insbesondere eine ökonomisch vorteilhafte Verfahrensführung und/oder einfache Automatisierbarkeit und/oder unproblematische Einhaltung der geforderten Fertigungstoleranzen.

Beim Flüssigspritzgießen wird ein flüssiges Polymermaterial verwendet, welches in der Regel aus verschiedenen Komponenten durch Mischen hergestellt wird. Bei den verschiedenen Komponenten kann es sich beispielsweise um eine Basis und eine katalysatorhaltige Komponente handeln. Wenn diese Komponenten miteinander vermischt werden, setzt eine Polymerisationsreaktion und/oder eine Vernetzungsreaktion ein. Anfangs ist die Mischung flüssig und kann durch Flüssigspritzgießen verarbeitet werden. Dazu wird die Mischung in die Gießformen dosiert. Das Material härtet in der Form aus und wird aus der Form ausgeworfen, wenn der Härtungsprozess ausreichend abgeschlossen ist. Aufgrund der flüssigen Verarbeitung ist das Flüssigspritzgießen apparativ weniger aufwendig als beispielsweise das HCR-Pressen, bei dem hohe Drücke und hohe Temperaturen angewandt werden müssen. Darüber hinaus ist die Verarbeitung mittels HCR-Pressen langsam.

Erfindungsgemäß handelt es sich bei dem durch Flüssigspritzgießen verarbeiteten Material um einen Silikonkautschuk (auch als "silicone rubber" bezeichnet). Das heißt, es wird zum Flüssigspritzgießen ein flüssiger Silikonkautschuk ("liquid silicone rubber" kurz "LSR") eingesetzt. Das elastische Material des Rückschlagventils 1 ist dann ein gehärteter und/oder vernetzter Flüssigsilikonkautschuk. Diese Bezeichnung bringt dabei nicht zum Ausdruck, dass das gehärtete und/oder vernetzte Material immer noch flüssig sei, sondern, dass es flüssig verarbeitet worden ist und daher die entsprechenden Eigenschaften aufweist.

In alternativen Ausführungsbeispielen handelt es sich bei dem durch Flüssigspritzgießen verarbeiteten Material um ein Polyolefin. In diesem Fall werden zum Flüssigspritzgießen vernetzungsfähige Polyolefin-Plastisole (auch als "PO-Plastisole" bezeichnet) verwendet.

Es ist bevorzugt, dass das elastische Material eine geringe Selbstheilungsrate aufweist, insbesondere eine geringe Selbstheilungsrate bei Einbringung von Energie in das Material, beispielsweise in Form von Wärme oder anderer Strahlung, beispielsweise einer zu Sterilisationszwecken eingebrachten energiereichen Strahlung (Gammastrahlung etc.).

Die Selbstheilungsrate eines elastischen Materials ist dabei definiert als der Anteil (in Prozent) einer Anzahl von mit einem Schnitt versehenen Probekörpern, deren Schnittflächen sich nach einer Testdauer zu maximal 10% durch teilweises Verschließen des Schlitzes verkleinert haben. Als Probekörper dienen dabei mindestens zehn flache Probekörper mit einer Dicke von 2 mm. Der Schnitt verläuft dabei in Richtung der Dicke des Probekörpers. Die Länge des Schnitts beträgt dabei 5 mm. Die Testdauer beträgt dabei 14 Tage. Während der Testdauer werden die Probekörper einer Temperatur von 50°C ausgesetzt.

In besonders bevorzugten Ausführungsbeispielen der Erfindung wird als Flüssigsilikonkautschuk ein Flüssigsilikonkautschuk mit vorteilhaften chemischen und physikalischen Eigenschaften ausgewählt, insbesondere ein Flüssigsilikonkautschuk, der ein elastisches Material mit niedriger Selbstheilungsrate ergibt und/oder einen geringen Gehalt an flüchtigen Substanzen aufweist und/oder den Anforderungen der für die Zulassung von Medizinprodukten zuständigen Behörden genügt.

Es ist ferner bevorzugt, dem durch Flüssigspritzgießen zu verarbeitenden Material vor oder während der Verarbeitung durch Flüssigspritzgießen ein Additiv zuzusetzen, um die Eigenschaften des elastischen Materials weiter zu verbessern. Das Additiv kann dabei mit jeder der Komponenten des durch Flüssigspritzgießen verarbeitbaren Materials zugesetzt sein oder es kann dem durch Flüssigspritzgießen verarbeitbaren Material als weitere Komponente zugesetzt werden. Beispielsweise kann ein geeignetes Additiv zugesetzt werden, um die Selbstheilungsrate zu erniedrigen.

Vorzugsweise wird das Additiv in einer Dosierung von mindestens 1 Gew-% und/oder höchstens 20 Gew-% zugesetzt. Die Gewichtsprozente (Gew.-%) ergeben sich dabei aus der Masse des Additivs im Verhältnis zur Summe der Masse des durch Flüssigspritzgießen zu verarbeitenden Materials und der Masse des Additivs. Mehr bevorzugt ist dabei eine Dosierung von mindestens 2 Gew.-% und höchstens 20 Gew.-%, am meisten bevorzugt von mindestens 2 Gew.-% und höchstens 10 Gew.-%. Dadurch lässt sich beispielsweise eine vorteilhaft niedrige Selbstheilungsrate bei gleichzeitig vorteilhaften elastischen Eigenschaften erzielen.

Das Additiv ist bevorzugt ein Füllstoff. Unter einem "Füllstoff" wird ein Additiv verstanden, welches sich im elastischen Matrixmaterial nicht oder nicht in wesentlichem Ausmaß löst.

Mehr bevorzugt handelt es sich bei dem als Additiv um einen Füllstoff, welcher Füllstoffpartikel beinhaltet oder aus diesen besteht. Dabei lösen sich die Füllstoffpartikel im elastischen Matrixmaterial weder während der Verarbeitung durch Flüssigspritzgießen noch danach auf und liegen daher im erfindungsgemäßen Rückschlagventil (1) selbst nach langer Lagerungszeit noch als in das Matrixmaterial dispergierte oder eingebettete Partikel vor. Ein derartiger Füllstoff kann dem elastischen Material beispielsweise in Form eines Pulvers zugesetzt werden. Das Pulver ist vorzugsweise ein Feinpulver, insbesondere in einer Größenordnung im Mikrometerbereich.

Beispielsweise kann es sich bei dem Füllstoff um einen der folgenden Stoffe oder um ein Gemisch eines der folgenden Stoffe oder um ein Gemisch mehrerer der folgenden Stoffe handeln:
- Glas, z.B. in Form von Glasfasern, Glaskugeln und Glasbruch
- mineralische Füllstoffe, z.B. Silikat oder Calciumcarbonat-Pulver
- Kohlenstofffasern
- Ruße.

Es ist dabei bevorzugt, dass das Additiv einen mineralischen Stoff umfasst, wobei es weiter bevorzugt ist, dass der mineralische Stoff ein Silikat ist. Insbesondere kann es sich bei dem mineralischen Füllstoff um Talkumpulver handeln.

Mit geeigneten Additiven, insbesondere mineralischen Füllstoffen und darunter bevorzugt Silikaten sowie besonders bevorzugt Talkum, kann es möglich sein, die Materialeigenschaften des durch Flüssigspritzgießen verarbeiteten Material zu verbessern. Unter einer Verbesserung der Materialeigenschaften wird dabei in erster Linie eine Erhöhung der Rauigkeit von Schnittflächen eines Schnitts im Material verstanden. Raue Schnittflächen liegen nicht plan aneinander, d.h. sie sind zumindest punktuell voneinander beabstandet. Dadurch sinkt die Neigung zur Selbstheilung.

Eine alternative Maßnahme zur Reduktion der Selbstheilung kann der Einsatz von Schmiermitteln als prozessbegleitende Hilfsstoffe sein. Schmiermittel bilden einen Schmierfilm auf den Schnittflächen, oder sieverhindern, dass sich die beiden gegenüberliegenden Schnittflächen ("Schlitzkanten") direkt berühren. Um dies zu erreichen, wird entweder beim Schlitzen des Ventilköpers ein flüssiges oder partikelförmiges Schmiermittel auf die Schnittflächen aufgetragen oder bereits dem Ausgangswerkstoff ein entsprechendes Öl oder Fett hinzugefügt, welches dann über die Lager- und Anwendungs-Dauer "ausschwitzt", d.h. nach und nach austritt. Diese alternative Maßnahme kann in der Umsetzung nicht nur relativ teuer sein. Durch die Schmiermittel kann es zudem zu einer Verunreinigung der Produktionsanlagen kommen, beispielsweise weil die Schmiermittel an Werkzeugen haften bleiben. Insbesondere ergeben sich Probleme, wenn austretendes Schmiermittel während der Zwischenlagerung der Rückschlagventile und während des Einbaus in Geräte (z.B. in medizinische Geräte) zu Verunreinigungen der Geräte sowie von Werkzeugen, Betriebsstätten, Verpackungsmaterialien etc. führt. Dadurch entsteht ein Mehraufwand durch Reinigungsmaßnahmen oder sogar eine Verschlechterung der Produktqualität. Problematisch sind austretende Hilfsstoffe insbesondere im medizinischen Bereich. Wenn diese Hilfsstoffe beispielsweise in eine Infusionslösung, die durch das Rückschlagventil fließt, gelangen, gelangen sie in der Folge in die Blutbahn eines Patienten.

Insbesondere vor diesem Hintergrund ist es bevorzugt, anstelle dieser alternativen Maßnahme dem durch Flüssigspritzgießen zu verarbeitenden Material vor oder während der Verarbeitung durch Flüssigspritzgießen wie oben beschrieben einen Füllstoff zuzusetzen, insbesondere einen mineralischen Füllstoff. Dadurch lassen sich erfindungsgemäße Silikonventile schnell, sauber und kostengünstig herstellen und gleichzeitig wird die Selbstheilungstendenz stark unterdrückt.

Während durch Flüssigspritzgießen aus elastischen Materialien, denen keine Füllstoffe zugesetzt sind, hergestellte Formteile üblicherweise transparent sind, ergeben sich beim Zusatz von Füllstoffen häufig opake Flüssigspritzguss-Formteile. Eine Opazität führt zu keiner Einschränkung der Gebrauchseigenschaften des erfindungsgemäßen Rückschlagventils.

Die Wahl des flüssigen Materials, von dem ausgehend das elastische Material durch Flüssigspritzgießen hergestellt wird, und/oder die Wahl von diesem Material zugesetzten Additiven ist ein wichtiger Aspekt von bevorzugten Ausführungsbeispielen, und kann zu besonders günstigen Materialeigenschaften führen. Besonders günstig sind dabei neben der Biokompatibilität und der Kompatibilität mit den Materialien der übrigen Komponenten eines Medizinprodukts beispielsweise eine Härte von mindestens 50 Shore A, eine besonders niedrige Selbstheilungsrate und die Möglichkeit, durch Schneiden des elastischen Materials einen Schnitt 7 mit Schnittflächen, die eine ausreichende Rauigkeit, insbesondere eine Rauigkeit von mindestens Ra = 0,15 µm, aufweisen.

In dem erfindungsgemäßen Verfahren zum Herstellen eines Rückschlagventils wird der Schritt des Durchschneidens des elastischen Materials entlang eines Schnitts bevorzugt so durchgeführt, dass die Schnittflächen eine ausreichende Rauigkeit, insbesondere eine Rauigkeit von mindestens Ra = 0,15 µm, aufweisen. Dies kann beispielsweise durch eine geeignete Wahl des Schneidwerkzeugs und/oder der Schneidgeschwindigkeit in Abhängigkeit vom jeweiligen elastischen Material erreicht werden.

Der Zusatz von Additiven, insbesondere von partikelhaltigen Hilfsstoffen, kann zu einer vorteilhaften, materialbedingten Rauigkeit der Schnittflächen, insbesondere eine Rauigkeit von mindestens Ra = 0,15 µm, beitragen oder dafür sorgen, dass die Schnittflächen eine derartige Rauigkeit aufweisen. Nach einer nicht einschränkenden Theorie liegt dies daran, dass im Bereich des Schnitts Partikel auftreten, die zumindest teilweise aus den Schnittflächen hervorstehen und dadurch eine Unebenheit der Schnittflächen ergeben.

## Patentansprüche

1. Rückschlagventil (1) umfassend einen topfförmigen Ventilkörper (2),
wobei der Ventilkörper (2) eine Mantelwand (3), eine Bodenwand (4) und eine der Bodenwand (4) gegenüberliegende Öffnung (5) aufweist,
wobei der Ventilkörper (2) ein elastisches Material umfasst,
wobei das elastische Material entlang eines Schnitts (7) durchschnitten ist,
wobei der Schnitt (7) in der Mantelwand angeordnet ist,
**dadurch gekennzeichnet, dass** das elastische Material ein durch Flüssigspritzgießen unter Verwendung von Flüssigsilikonkautschuk verarbeitetes Material umfasst,
wobei der Schnitt (7)
entweder parallel zur Innenfläche (41) der Bodenwand (4) ist, oder nicht parallel zur Innenfläche (41) der Bodenwand (4) geneigt ist und dabei eine Neigung zur Innenfläche (41) der Bodenwand (4) aufweist, die maximal 10°, vorzugsweise maximal 5°, vorzugsweise maximal 2°, insbesondere maximal 1° beträgt.

2. Rückschlagventil (1) gemäß Anspruch 1,
wobei das durch Flüssigspritzgießen verarbeitete Material eine Selbstheilungsrate von maximal 50%, bevorzugt maximal 20%, mehr bevorzugt maximal 10%, noch mehr bevorzugt maximal 5%, am meisten bevorzugt maximal 1% aufweist.

3. Rückschlagventil (1) gemäß einem der vorstehenden Ansprüche,
wobei das elastische Material ein Additiv umfasst,
wobei das Additiv bevorzugt ein Füllstoff ist,
wobei der Füllstoff weiter bevorzugt einen mineralischen Stoff umfasst und/oder wobei der Füllstoff weiter bevorzugt Partikel umfasst,
wobei der mineralische Stoff noch weiter bevorzugt ein Silikat ist,
wobei der mineralische Stoff am meisten bevorzugt Talkumpulver ist.

4. Rückschlagventil gemäß Anspruch 3,
wobei das Additiv einen funktionalen Füllstoff umfasst, welcher eine Rauigkeit einer Schnittfläche des Schnitts im Vergleich zu einem durch Flüssigspritzgießen verarbeiteten Material ohne diesen Füllstoff erhöht,
wobei die Rauigkeit der Schnittfläche des Schnitts im Vergleich zu einem durch Flüssigspritzgießen verarbeiteten Material ohne diesen Füllstoff bevorzugt um mindestens 10%, mehr bevorzugt mindestens 25%, noch mehr bevorzugt um mindestens 50% erhöht ist.

5. Rückschlagventil (1) gemäß einem der vorstehenden Ansprüche,
wobei die Mantelwand (3) und die Bodenwand (4) einstückig ausgebildet sind.

6. Rückschlagventil (1) gemäß einem der vorstehenden Ansprüche,
wobei an der Außenfläche der Mantelwand (3) eine Wulst (32) und/oder eine Nut vorgesehen ist, und /oder
wobei an der Innenfläche der Mantelwand (3) eine Wulst und/oder eine Nut vorgesehen ist.

7. Rückschlagventil (1) gemäß einem der vorstehenden Ansprüche,
wobei sich die Innenfläche der Mantelwand (3) in Richtung zur Bodenwand (4) verjüngt, bevorzugt konisch verjüngt und/oder
wobei an die Mantelwand (3) ein die Öffnung (5) umgebender Flansch (6) angeformt ist, wobei bevorzugt die Mantelwand (3) und der Flansch (6) einstückig ausgebildet sind.

8. Rückschlagventil (1) gemäß einem der vorstehenden Ansprüche,
wobei der Schnitt (7) Schnittflächen aufweist, welche eine Rauigkeit von mindestens Ra=0,15 µm, bevorzugt mindestens Ra=0,20 µm, mehr bevorzugt mindestens Ra=0,25 µm aufweist.

9. Tropfkammer für ein Infusions- oder Transfusionssystem,
wobei die Tropfkammer ein Belüftungsventil aufweist und
wobei als Belüftungsventil ein Rückschlagventil (1) gemäß einem der Ansprüche 1 bis 8 dient.

10. Port zum nadellosen Zudosieren einer Flüssigkeit in ein Infusions- oder Transfusionssystem, wobei als Port ein Rückschlagventil (1) gemäß einem der Ansprüche 1 bis 8 dient.

11. Rückflusssperre für die Fluidleitung eines Infusions- oder Transfusionssystems, wobei als Rückflusssperre ein Rückschlagventil (1) gemäß einem der Ansprüche 1 bis 8 dient.

12. Infusions- oder Transfusionssystem mit mindestens einer Komponente ausgewählt aus
- Tropfkammer, wobei als Tropfkammer eine Tropfkammer gemäß Anspruch 9 dient,
- Port zum nadellosen Zudosieren einer Flüssigkeit, wobei als Port ein Port gemäß Anspruch 10 dient,
- Rückflusssperre zum Verhindern eines Flusses eines Fluids in einer Fluidleitung in einer Fließrichtung, wobei als Rückflusssperre eine Rückflusssperre gemäß Anspruch 11 dient.

13. Verfahren zum Herstellen eines Rückschlagventils (1), gemäß einem der Ansprüche 1-8, welches einen topfförmigen Ventilkörper (2) mit einer Mantelwand (3), einer Bodenwand (4) und einer der Bodenwand (4) gegenüberliegenden Öffnung (5) aufweist, wobei das Verfahren die folgenden Schritte umfasst:
(A) Bereitstellen eines elastischen Materials umfassend Flüssigsilikonkautschuk,
(B) Flüssigspritzgießen des elastischen Materials zum Herstellen des Ventilkörpers (2),
(C) Durchschneiden des elastischen Materials entlang eines Schnitts (7), wobei der Schnitt (7) in der Mantelwand (3) angeordnet ist, und
entweder parallel zur Innenfläche (41) der Bodenwand (4) ist, oder nicht parallel zur Innenfläche (41) der Bodenwand (4) geneigt ist und dabei eine Neigung zur Innenfläche (41) der Bodenwand (4) aufweist, die maximal 10°, vorzugsweise maximal 5°, vorzugsweise maximal 2°, insbesondere maximal 1° beträgt.

14. Verfahren gemäß Anspruch 13,
wobei vor Schritt (A) und/oder zwischen Schritt (A) und Schritt (B) und/oder während Schritt (C) dem elastischen Material ein Additiv zugesetzt wird,
wobei bevorzugt die Dosierung des Additivs mindestens 1 Gew.-%, mehr bevorzugt 2 Gew.-% und /oder höchstens 20 Gew.-%, mehr bevorzugt höchstens 10 Gew.-% bezogen auf die Gesamtmenge aus Additiv und elastischem Material beträgt, und/oder
wobei das Additiv bevorzugt ein Füllstoff ist,
wobei der Füllstoff weiter bevorzugt einen mineralischen Stoff umfasst und/oder wobei der Füllstoff weiter bevorzugt Partikel umfasst,
wobei der mineralische Stoff noch weiter bevorzugt ein Silikat ist,
wobei der mineralische Stoff am meisten bevorzugt Talkumpulver ist.

## Claims

1. Check valve (1) comprising a pot-shaped valve body (2),
the valve body (2) having an outer wall (3), a bottom wall (4) and an opening (5) opposite the bottom wall (4),
the valve body (2) comprising an elastic material,
the elastic material being cut along a cut (7), the cut (7) being arranged in the outer wall,
**characterized in that** the elastic material comprises a material processed by liquid injection molding using liquid silicone rubber,
the cut (7) being either parallel to the inner surface (41) of the bottom wall (4) or being inclined not parallel to the inner surface (41) of the bottom wall (4) and having an inclination to the inner surface (41) of the bottom wall (4) of at most 10°, preferably at most 5°, preferably at most 2°, in particular at most 1°.

2. Check valve (1) according to claim 1,
wherein the material processed by liquid injection molding has a self-healing rate of at most 50%, preferably at most 20%, more preferably at most 10%, even more preferably at most 5%, most preferably at most 1%.

3. Check valve (1) according to either of the preceding claims,
wherein the elastic material comprises an additive,
wherein the additive is preferably a filler,
wherein the filler more preferably comprises a mineral substance and/or wherein the filler more preferably comprises particles,
wherein the mineral substance is even more preferably a silicate,
wherein the mineral substance is most preferably talc powder.

4. Check valve according to claim 3,
wherein the additive comprises a functional filler which increases the roughness of a cut surface of the cut compared to a material processed by liquid injection molding without this filler,
wherein the roughness of the cut surface of the cut is increased preferably by at least 10%, more preferably by at least 25%, even more preferably by at least 50%, compared to a material processed by liquid injection molding without this filler.

5. Check valve (1) according to any of the preceding claims,
wherein the outer wall (3) and the bottom wall (4) are formed in one piece.

6. Check valve (1) according to any of the preceding claims,
wherein a bead (32) and/or a groove is provided on the outer surface of the outer wall (3), and/or
wherein a bead and/or a groove is provided on the inner surface of the outer wall (3).

7. Check valve (1) according to any of the preceding claims,
wherein the inner surface of the outer wall (3) tapers toward the bottom wall (4), preferably tapering conically and/or
wherein a flange (6) surrounding the opening (5) is integrally formed on the outer wall (3), wherein preferably the outer wall (3) and the flange (6) are formed in one piece.

8. Check valve (1) according to any of the preceding claims,
wherein the cut (7) has cut surfaces which have a roughness of at least Ra=0.15 µm, preferably at least Ra=0.20 µm, more preferably at least Ra=0.25 µm.

9. Drip chamber for an infusion or transfusion system,
wherein the drip chamber has a ventilation valve and
wherein a check valve (1) according to any of claims 1 to 8 serves as the ventilation valve.

10. Port for needle-free dosing of a liquid into an infusion or transfusion system, wherein a check valve (1) according to any of claims 1 to 8 serves as the port.

11. Backflow prevention device for the fluid line of an infusion or transfusion system, wherein a check valve (1) according to any of claims 1 to 8 serves as the backflow prevention device.

12. Infusion or transfusion system comprising at least one component selected from
- drip chamber, wherein a drip chamber according to claim 9 serves as the drip chamber,
- port for needle-free dosing of a liquid, wherein a port according to claim 10 serves as the port,
- backflow prevention device for preventing the flow of a fluid in a fluid line in one flow direction, wherein a backflow prevention device according to claim 11 serves as the backflow prevention device.

13. Method for producing a check valve (1) according to any of claims 1-8, which comprises a pot-shaped valve body (2) having an outer wall (3), a bottom wall (4) and an opening (5) opposite the bottom wall (4),
wherein the method comprises the following steps:
(A) providing an elastic material comprising liquid silicone rubber,
(B) liquid injection molding of the elastic material to produce the valve body (2),
(C) cutting the elastic material along a cut (7), wherein the cut (7) is arranged in the outer wall (3), and
is either parallel to the inner surface (41) of the bottom wall (4), or is inclined not parallel to the inner surface (41) of the bottom wall (4) and has an inclination to the inner surface (41) of the bottom wall (4) of at most 10°, preferably at most 5°, preferably at most 2°, in particular at most 1°.

14. Method according to claim 13,
wherein an additive is added to the elastic material before step (A) and/or between step (A) and step (B) and/or during step (C),
wherein preferably the dosage of the additive is at least 1 wt.%, more preferably 2 wt.% and/or at most 20 wt.%, more preferably at most 10 wt.%, based on the total amount of additive and elastic material, and/or
wherein the additive is preferably a filler,
wherein the filler more preferably comprises a mineral substance and/or wherein the filler more preferably comprises particles,
wherein the mineral substance is even more preferably a silicate,
wherein the mineral substance is most preferably talc powder.

## Revendications

1. Soupape antiretour (1) comprenant un corps de soupape (2) en forme de pot,
dans laquelle le corps de soupape (2) présente une paroi enveloppante (3), une paroi de fond (4) et une ouverture (5) opposée à la paroi de fond (4),
dans laquelle le corps de soupape (2) comprend un matériau élastique,
dans laquelle le matériau élastique est découpé le long d'une découpe (7), dans laquelle la découpe (7) est disposée dans la paroi enveloppante,
**caractérisée en ce que** le matériau élastique comprend un matériau façonné par moulage par injection de liquide à l'aide de caoutchouc de silicone liquide,
dans laquelle la découpe (7) est soit parallèle à la surface intérieure (41) de la paroi de fond (4), soit non parallèle à la surface intérieure (41) de la paroi de fond (4), et présente alors une inclinaison par rapport à la surface intérieure (41) de la paroi de fond (4) qui est de 10° au maximum, de préférence de 5° au maximum, de préférence de 2° au maximum, en particulier de 1° au maximum.

2. Soupape antiretour (1) selon la revendication 1,
dans laquelle le matériau façonné par moulage par injection de liquide présente un taux d'autoguérison de 50 % au maximum, de préférence de 20 % au maximum, plus préférablement de 10 % au maximum, encore plus préférablement de 5 % au maximum, le plus préférablement de 1 % au maximum.

3. Soupape antiretour (1) selon l'une des revendications précédentes,
dans laquelle le matériau élastique comprend un additif,
dans laquelle l'additif est de préférence une charge,
dans laquelle la charge comprend plus préférablement une substance minérale et/ou dans laquelle la charge comprend plus préférablement des particules,
dans laquelle la substance minérale est encore plus préférablement un silicate,
dans laquelle la substance minérale est le plus préférablement de la poudre de talc.

4. Soupape antiretour selon la revendication 3,
dans laquelle l'additif comprend une charge fonctionnelle qui augmente la rugosité d'une surface de découpe de la découpe par rapport à celle d'un matériau façonné par moulage par injection de liquide sans ladite charge,
dans laquelle la rugosité de la surface de découpe de la découpe est de préférence augmentée d'au moins 10 %, plus préférablement d'au moins 25 %, encore plus préférablement d'au moins 50 %, par rapport à celle d'un matériau façonné par moulage par injection de liquide sans ladite charge.

5. Soupape antiretour (1) selon l'une des revendications précédentes,
dans laquelle la paroi enveloppante (3) et la paroi de fond (4) sont réalisées d'un seul tenant.

6. Soupape antiretour (1) selon l'une des revendications précédentes,
dans laquelle un renflement (32) et/ou une rainure sont prévus sur la surface extérieure de la paroi enveloppante (3), et/ou
dans laquelle un renflement et/ou une rainure sont prévus sur la surface intérieure de la paroi enveloppante (3).

7. Soupape antiretour (1) selon l'une des revendications précédentes,
dans laquelle la surface intérieure de la paroi enveloppante (3) se rétrécit en direction de la paroi de fond (4), de préférence se rétrécit de manière conique, et/ou
dans laquelle une bride (6) entourant l'ouverture (5) est moulée sur la paroi enveloppante (3), dans laquelle, de préférence, la paroi enveloppante (3) et la bride (6) sont réalisées d'un seul tenant.

8. Soupape antiretour (1) selon l'une des revendications précédentes,
dans laquelle la découpe (7) présente des surfaces de découpe dont la rugosité est d'au moins Ra = 0,15 µm, de préférence d'au moins Ra = 0,20 µm, plus préférablement d'au moins Ra = 0,25 µm.

9. Chambre compte-gouttes pour un système de perfusion ou de transfusion,
dans laquelle la chambre compte-gouttes présente une soupape d'aération et
dans laquelle une soupape antiretour (1) selon l'une des revendications 1 à 8 sert de soupape d'aération.

10. Orifice pour l'administration dosée sans aiguille d'un liquide dans un système de perfusion ou de transfusion, dans lequel une soupape antiretour (1) selon l'une des revendications 1 à 8 sert d'orifice.

11. Dispositif antiretour pour la conduite de fluide d'un système de perfusion ou de transfusion, dans lequel une soupape antiretour (1) selon l'une des revendications 1 à 8 sert de dispositif antiretour.

12. Système de perfusion ou de transfusion comportant au moins un composant choisi parmi
- une chambre compte-gouttes, dans lequel une chambre compte-gouttes selon la revendication 9 sert de chambre compte-gouttes,
- un orifice pour l'administration dosée sans aiguille d'un liquide, dans lequel un orifice selon la revendication 10 sert d'orifice,
- un dispositif antiretour permettant d'empêcher l'écoulement d'un fluide dans une conduite de fluide dans un sens d'écoulement, dans lequel un dispositif antiretour selon la revendication 11 sert de dispositif antiretour.

13. Procédé pour la fabrication d'une soupape antiretour (1), selon l'une des revendications 1 à 8, laquelle présente un corps de soupape (2) en forme de pot comportant une paroi enveloppante (3), une paroi de fond (4) et une ouverture (5) opposée à la paroi de fond (4),
dans lequel le procédé comprend les étapes suivantes :
(A) fourniture d'un matériau élastique comprenant du caoutchouc de silicone liquide,
(B) moulage par injection de liquide du matériau élastique pour la fabrication du corps de soupape (2),
(C) découpe du matériau élastique le long d'une découpe (7), dans lequel la découpe (7) est disposée dans la paroi enveloppante (3), et
soit est parallèle à la surface intérieure (41) de la paroi de fond (4), soit n'est pas parallèle à la surface intérieure (41) de la paroi de fond (4), et présente alors une inclinaison par rapport à la surface intérieure (41) de la paroi de fond (4) qui est de 10° au maximum, de préférence de 5° au maximum, de préférence de 2° au maximum, en particulier de 1° au maximum.

14. Procédé selon la revendication 13,
dans lequel un additif est ajouté au matériau élastique avant l'étape (A) et/ou entre l'étape (A) et l'étape (B) et/ou pendant l'étape (C),
dans lequel, de préférence, la dose de l'additif est d'au moins 1 % en poids, plus préférablement de 2 % en poids et/ou de 20 % en poids au maximum, plus préférablement de 10 % en poids au maximum, par rapport à la quantité totale d'additif et de matériau élastique, et/ou
dans lequel l'additif est de préférence une charge,
dans lequel la charge comprend plus préférablement une substance minérale et/ou dans lequel la charge comprend plus préférablement des particules,
dans lequel la substance minérale est encore plus préférablement un silicate,
dans lequel la substance minérale est le plus préférablement de la poudre de talc.
